# EUROPEAN PATENT SPECIFICATION

(11) **EP 2 934 304 B1**
(45) Date of publication and mention of the grant of the patent: **13.10.2021**
(21) Application number: 13863844.0
(22) Date of filing: 18.12.2013
(51) Int. Cl.: A61B 5/02, A61B 8/06, A61B 8/12, A61B 8/00, A61B 5/0215, A61B 5/026, A61B 8/08

(54) **MULTI-SENSOR DEVICES**
MULTISENSORVORRICHTUNGEN
DISPOSITIFS À PLUSIEURS CAPTEURS

(30) Priority: 21.12.2012 US 201261745001 P
(43) Date of publication of application: 28.10.2015
(73) Proprietor: Philips Image Guided Therapy Corporation, San Diego CA 92130 (US)
(72) Inventor: ANDERSON David, San Diego, CA 92130 (US)
(74) Representative: de Haan, Poul Erik
(86) International application number: PCT/US2013/076053
(87) International publication number: WO 2014/100140

(56) References cited:
- WO-A1-2012/061935
- WO-A1-2012/061935
- WO-A1-2012/155040
- WO-A1-2012/155040
- WO-A1-2012/166332
- US-A1- 2002 041 723
- US-A1- 2008 114 254
- US-A1- 2011 137 140
- US-A1- 2011 137 140
- US-A1- 2012 108 943
- US-A1- 2012 108 943
- US-A1- 2012 220 883
- US-A1- 2012 238 869
- None

## Description

### Technical Field

The present invention generally relates to devices that include multiple sensors for measuring different characteristics inside a vessel. For example, devices of the invention may have sensors for imaging the interior of a vessel while also detecting both pressure and/or detecting flow inside the vessel.

### Background

Cardiovascular disease frequently arises from the accumulation of atheromatous deposits on inner walls of vascular lumen, particularly the arterial lumen of the coronary and other vasculature, resulting in a condition known as atherosclerosis. These deposits can have widely varying properties, with some deposits begin relatively soft and others being fibrous and/or calcified. In the latter case, the deposits are frequently referred to as plaque. These deposits can restrict blood flow, which in severe cases can lead to myocardial infarction.

The assessment of cardiovascular disease often involves assessing the condition of the vessel and the accumulation of plaque using a variety of techniques. These techniques include intravascular ultrasound (IVUS) imaging, in which sound waves are used to image the interior of a blood vessel. In IVUS, an imaging catheter is typically threaded over a guidewire into a blood vessel, and images of the atherosclerotic plaque and surrounding area are acquired using ultrasonic echoes.

Additional methods for assessing the condition of the vessel include determining the relative pressure within the vessel and the speed at which blood moves through the vessel, also known as flow. These methods typically involve the use of a guidewire inserted to blood vessel to measure such parameters, which are subsequently used to determine other criteria, such as Fractional Flow Reserve (FFR). FFR is a measurement of the maximum myocardial flow in the presence of a stenosis (i.e., a narrowing of the blood vessel) divided by the normal maximum myocardial flow. This ratio is approximately equal to the mean hyperemic (i.e., dilated vessel) distal coronary pressure divided by the mean arterial pressure. An FFR below a certain value typically indicates that therapeutic intervention is required.

One problem with these assessment techniques is that they typically involve the use of a number of devices to perform various functions, i.e., an imaging catheter for performing IVUS, a pressure sensing guidewire to determine blood pressure, and a flow sensing guidewire to determine blood velocity. Even in the best of cases, conventional devices permit the combination of pressure and flow detection on a single guidewire, however, IVUS is still confined to a separate device, such as an imaging catheter. The need for several devices complicates and undesirably lengthens the interventional procedure and further delays subsequent treatment.

WO 2012/166332 A1 discloses a probe comprising an optical coherence tomography assembly and a pressure assembly.

US 2012/108943 A1 discloses a fiber Bragg Grating (FBG) photoacoustic transducer integrated in an imaging guidewire, the transducer being configured as both an ultrasound imaging sensor and an ultrasound blood flow sensor.

### Summary

The present invention is defined in the claims, and relates to devices that include multiple sensors for measuring different characteristics inside a vessel. The devices provide ultrasound imaging in the interior of a vessel while also providing ultrasound flow and pressure sensing. Such devices are guidewires having pressure, flow, and imaging sensors positioned thereon. The combination of multiple elements into one device significantly reduces the complexity of cardiovascular assessment procedures by eliminating the need to use separate devices to accomplish different tasks. Moreover, devices of the invention combine imaging and measurement technologies in order to evaluate the need for therapy and for the successful administration of therapy. With one device, a physician can, for example, image the interior of a vessel using IVUS, measure pressure inside a vessel, and determine the flow of blood inside the vessel. The physician can then slide, for example, a stent-delivering catheter over the provided guidewire, place the stent, and subsequently evaluate placement using the provided device.

According to the invention, the imaging element, pressure detecting element, and flow detecting elements are contained in a single optical fiber mounted inside a hollow guidewire. Rather than utilizing a separate pressure sensor, flow sensor, and imaging element, the optical fiber provides a base from which all three elements can operate. Accordingly, the encompassed devices can be smaller than conventional devices as well as easier to construct.

For the detection and imaging elements that are fiber-optic based, a variety of configurations are available to facilitate the operation of these elements. For example, the pressure sensing element may include a Fabry-Perot type cavity, in which two partially reflective surfaces are aligned with each other such that many waves of light derived from the same incident wave can interfere. The resultant interference patterns may be used to analyze the spectral character of the incident beam.

The provided devices may also incorporate opto-acoustic methods, in which optical energy is converted into acoustic energy that can be used to image the interior of a blood vessel. In certain aspects of the invention, blazed Fiber Bragg Gratings (FBGs) are used to obliquely deliver sound waves relative to the guidewire. In other aspects of the invention, blazed FBGs are not used to emit the ultrasonic signal. Instead, a dual core optical fiber may be used to refract light at incident angles in order to eventually emit an ultrasonic signal.

The flow sensor can use the same signal path and device components as the imaging sensor, but different signal processing techniques are used depending on the desired function. For example, a longer acoustic signal may be used for flow determination, rather than the short pulse used for imaging. Typical Doppler flow signal processing may then be used to measure the phase shift of the reflected ultrasound. Because the same elements can be used for both imaging and flow detection, in certain aspects of the invention, the imaging sensor and the flow sensor comprise a single sensor that can be used for either function.

The device is able to image, measure pressure, and determine flow, thereby providing an assessment of the vessel condition. The incorporation of optical elements into the device allows one device to perform all these functions. With such a device, the provided methods significantly expedite the diagnosis of cardiovascular disease and provide a more thorough assessment when compared to conventional methods that use single function devices.

### Brief Description of Drawings

FIG. 1 depicts an optical fiber suitable for use with the provided imaging devices.
FIG. 2 depicts an example not forming part of the invention of an imaging element that includes a piezoelectric element.
FIGS. 3 and 4 depict an imaging element that uses Fiber Bragg Gratings to generate acoustic energy.
FIG. 5 is a block diagram generally illustrating an image assembly and several associated interface components.
FIG. 6 is a block diagram illustrating another example of an imaging assembly and associated interface components
FIG. 7 illustrates an exemplary optical fiber configuration for use in practicing the invention
FIG. 8 illustrates a second exemplary optical fiber configuration for use in practicing the invention.
FIG. 9 illustrates an exemplary guidewire configuration for use in practicing the invention.
FIG. 10 illustrates an exemplary guidewire configuration for use in practicing the invention from a close-up, cross-sectional perspective.

### Detailed Description

The present invention generally relates to devices that include multiple sensors for measuring different characteristics inside a vessel and can not only image the inside of a vessel, but can also measure pressure and flow inside a vessel. The device can include an elongate body configured for insertion into a vessel, a pressure sensor positioned on the elongate body configured to detect pressure in the interior of the vessel, an imaging sensor positioned on the elongate body configured to image the interior of the vessel, and a flow sensor positioned on the elongate body configured to detect the velocity of a liquid moving within the interior of the vessel. The devices provided herein provide greater benefit to a physician by combining multiple features into one product.

The devices of the present invention encompasse an guidewire. The guidewire is configured for intraluminal introduction into a target body lumen. The dimensions and other physical characteristics of the catheter or guidewire may vary depending on the body lumen that is to be accessed. In addition, the dimensions can depend on the placement and number of imaging elements included on the imaging catheter or guidewire.

The provided imaging catheters and guidewires may also serve other functions in addition to imaging. In certain aspects, the provided imaging catheter and/or guidewire may also serve as a delivery catheter and/or guidewire for delivery of some type of a therapeutic device, such as a stent, ablator, or balloon. During the procedure, the imaging catheter may be used to identify the appropriate location and the delivery catheter used to deliver the device to the appropriate location. In certain embodiments, the provided imaging guidewire may serve as rail for the introduction of a catheter. The catheter is slid over the provided guidewire and used as normal.

For embodiments encompassing an imaging guidewire, the imaging element can be formed as or be integrated into the body of the imaging guidewire, circumscribe the guidewire, and/or run along the body of the guidewire. The imaging guidewire may also include an outer support structure or coating surrounding the imaging elements. The imaging guidewire including the imaging element (for example, an optical fiber and transducer material) and, in certain embodiments, the surrounding support structure can have a total outside diameter of less than 1 mm, preferably less than 300 micron (less than about 1 French).

The provided imaging guidewire bodies may include a solid metal or polymer core. Suitable polymers include polyvinylchloride, polyurethanes, polyesters, polytetrafluoroethylenes (PTFE), silicone rubbers, natural rubbers, and the like. Preferably, at least a portion of the metal or polymer core and other elements that form the imaging guidewire body are flexible.

In certain embodiments, an imaging catheter is provided. The imaging element can form or be integrated within the body of the catheter, circumscribe the catheter, placed on a distal end face of the catheter, and/or run along the body of the catheter. The imaging catheter may also include an outer support structure or coating surrounding the imaging elements. Imaging catheter bodies intended for intravascular introduction will typically have a length in the range from 50 cm to 200 cm and an outer diameter in the range from 1 French to 12 French (0.33 mm: 1 French), usually from 3 French to 9 French. In the case of coronary catheters, the length is typically in the range from 125 cm to 200 cm, the diameter is preferably below 8 French, more preferably below 7 French, and most preferably in the range from 2 French to 7 French.

Catheter bodies will typically be composed of an organic polymer that is fabricated by conventional extrusion techniques. Suitable polymers include polyvinylchloride, polyurethanes, polyesters, polytetrafluoroethylenes (PTFE), silicone rubbers, natural rubbers, and the like. Optionally, the catheter body may be reinforced with braid, helical wires, coils, axial filaments, or the like, in order to increase rotational strength, column strength, toughness, pushability, and the like. Suitable catheter bodies may be formed by extrusion, with one or more channels being provided when desired. The catheter diameter can be modified by heat expansion and shrinkage using conventional techniques. The resulting catheters will thus be suitable for introduction to the vascular system, often the coronary arteries, by conventional techniques. Preferably, at least a portion of the catheter body is flexible.

The imaging guidewire of the invention includes an imaging assembly such as optical-acoustic imaging apparatus. The imaging assembly is used to send and receive signals to and from the imaging surface that form the imaging data.

In preferred embodiments, the imaging assembly is configured to send and receive an optical/light signal through an imaging element of the device. The imaging element comprises an optical fiber through which the imaging assembly sends and receives optical signals. In certain embodiments, the optical fiber includes a Fiber Bragg Grating within the optical fiber. Further detail regarding the imaging elements is provided throughout the present disclosure.

Fiber Bragg Gratings (FBGs) provide a means for measuring the interference between two paths taken by an optical beam. A partially-reflecting Fiber Bragg Grating is used to split the incident beam of light into two parts, in which one part of the beam travels along a path that is kept constant (constant path) and another part travels a path for detecting a change (change path). The paths are then combined to detect any interference in the beam If the paths are identical, then the two paths combine to form the original beam. If the paths are different, then the two parts will add or subtract from each other and form an interference. The Fiber Bragg Grating elements are thus able to sense a change wavelength between the constant path and the change path based on received ultrasound or acoustic energy. The detected optical signal interferences can be used to generate an image using any conventional means.

In certain examples not forming part of the invention, the imaging element includes a piezoelectric element to generate the acoustic or ultrasound energy. In such aspect, the optical fiber of the imaging element may by coated by the piezoelectric element. The piezoelectric element may include any suitable piezoelectric or piezoceramic material. In one embodiment, the piezoelectric element is a poled polyvinylidene fluoride or polyvinylidene difluoride material. The piezoelectric element can be connected to one or more electrodes that are connected to a generator that transmits pulses of electricity to the electrodes. The electric pulses cause mechanical oscillations in the piezoelectric element, which generates an acoustic signal. Thus, the piezoelectric element is an electric-to-acoustic transducer. Primary and reflected pulses (i.e. reflected from the imaging medium) are received by the Bragg Grating element and transmitted to an electronic instrument to generate an image.

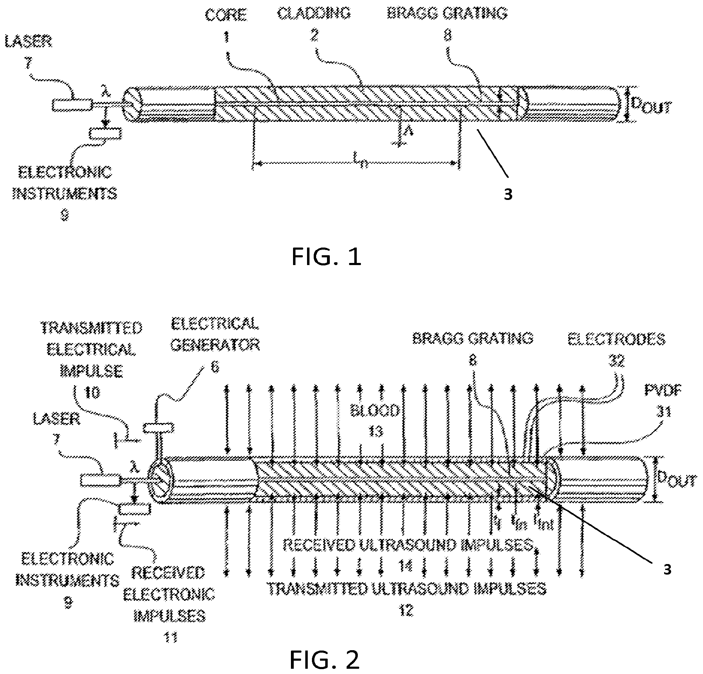

FIG. 2 depicts an example not forming part of the invention of an imaging element that includes a piezoelectric element. The imaging element includes an optical fiber 3 (such as the optical fiber in FIG. 1) with Fiber Bragg Grating 8 and a piezoelectric element 31. As shown in FIG. 2, an electrical generator 6 stimulates the piezoelectric element 31 (electrical-to-acoustic transducer) to transmit ultrasound impulses 10 to both the Fiber Bragg Grating 8 and the outer medium 13 in which the device is located. For example, the outer medium may include blood when imaging a vessel. Primary and reflected impulses 11 are received by the Fiber Bragg Grating 8 (acting as an acoustic-to-optical transducer). The mechanical impulses deform the Bragg Grating and cause the Fiber Bragg Grating to modulate the light reflected within the optical fiber, which generates an interference signal. The interference signal is recorded by electronic detection instrument 9, using conventional methods. The electronic instrument may include a photodetector and an oscilloscope. Imaging information regarding the contact between the imaging device and the object can be generated from these recorded signals. The electronic instruments 9 modulation of light reflected backwards from the optical fiber due to mechanical deformations. The optical fiber with a Bragg Grating described herein and shown in FIG. 1, the imaging element described herein and shown in FIG. 2 and other varying embodiments are described in more detail in U.S. Patent Nos. 6,659,957 and 7,527,594 and in U.S. Patent Publication No. 2008/0119739.

In another aspect, the imaging element does not require an electrical-to-acoustic transducer to generate acoustic/ultrasound signals. Instead, the imaging element utilizes the one or more Fiber Bragg Grating elements of the optical fiber in combination with an optical-to-acoustic transducer material to generate acoustic energy from optical energy. In this aspect, the acoustic-to-optical transducer (signal receiver) also acts as an optical-to-acoustic transducer (signal generator).

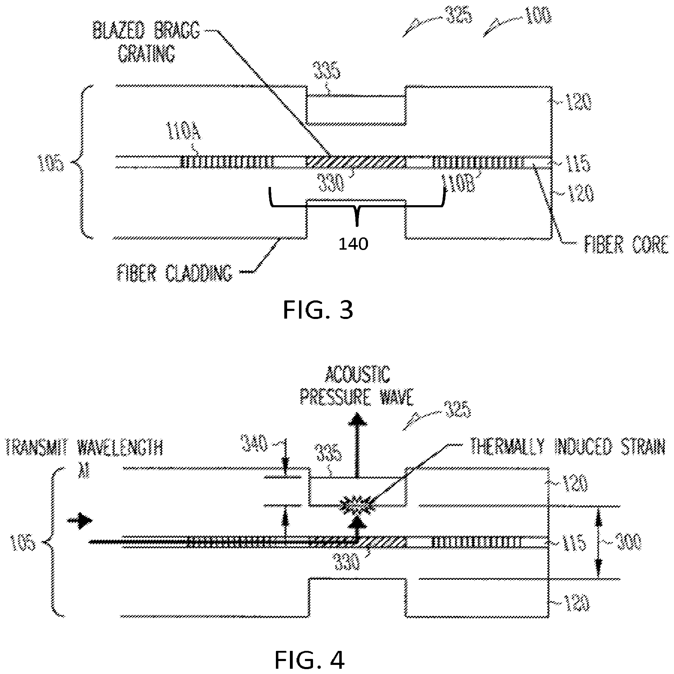

To generate the acoustic energy, imaging element may include a combination of blazed and unblazed Fiber Bragg Gratings. Unblazed Bragg Gratings typically include impressed index changes that are substantially perpendicular to the longitudinal axis of the fiber core of the optical fiber. Unblazed Bragg Gratings reflect optical energy of a specific wavelength along the longitudinal of the optical fiber. Blazed Bragg Gratings typically include obliquely impressed index changes that are at a non-perpendicular angle to the longitudinal axis of the optical fiber. Blazed Bragg Gratings reflect optical energy away from the longitudinal axis of the optical fiber. FIGS. 3 and 4 depict an imaging element according to this embodiment.

FIG. 3 shows an example of an imaging element that uses Fiber Bragg Gratings to generate acoustic energy. As depicted in FIG. 4, the imaging element 100 includes an optical fiber 105 with unblazed Fiber Bragg Grating IlOA and HOB and blazed Fiber Bragg Grating 330 and a photoacoustic material 335 (optical-to-acoustic transducer). The region between the unblazed Fiber Bragg Grating IlOA and HOB is known as the strain sensing region 140. The strain sensing region may be, for example, 1 mm in length. The Blazed Fiber Bragg Grating is implemented in the strain sensing region 140. The photoacoustic material 335 is positioned to receive the reflected optical energy from the blazed Fiber Bragg Grating 330. Although not shown, the proximal end of the optical fiber 105 is operably coupled to a laser and one or more electronic detection elements.

In operation and as depicted in FIG. 4, the blazed Fiber Bragg Grating 330 receives optical energy of a specific wavelength λ1 from a light source, e.g. a laser, and blazed Grating 330 directs that optical energy towards photoacoustic material 335. The received optical energy in the photoacoustic material 335 is converted into heat, which causes the material 335 to expand. Pulses of optical energy sent to the photoacoustic material 335 cause the photoacoustic material 335 to oscillate. The photoacoustic material 335 oscillates, due to the received optical energy, at a pace sufficient to generate an acoustic or ultrasound wave. The acoustic wave is transmitted out to and reflected from the object surface back to the imaging element, particularly when the device contacts an object. The acoustic wave reflected from the object surface impinges on photoacoustic transducer 335, which causes a vibration or deformation of photoacoustic transducer 335. This results in a change in length of light path within the strain sensing region 140. Light received by blazed fiber Bragg grating from photoacoustic transducer 135 and into fiber core 115 combines with light that is reflected by either fiber Bragg grating 110A or 110B (either or both may be including in various embodiments). The light from photoacoustic transducer 135 will interfere with light reflected by either fiber Bragg grating 110A or 110B and the light returning to the control unit will exhibit an interference pattern. This interference pattern encodes the image captured by imaging element 100. The light 137 can be received into photodiodes within a control unit and the interference pattern thus converted into an analog electric signal. This signal can then be digitized using known digital acquisition technologies and processed, stored, or displayed as an image of the target treatment site.

Acoustic energy of a specific frequency may be generated by optically irradiating the photoacoustic material 335 at a pulse rate equal to the desired acoustic frequency. The photoacoustic material 335 can be any suitable material for converting optical energy to acoustic energy and any suitable thickness to achieve a desired frequency. The photoacoustic material 335 may have a coating or be of a material that receives acoustic energy over a band of frequencies to improve the generation of acoustic energy by the photoacoustic material and reception of the acoustic energy by the optical fiber imaging region.

In one example, the photoacoustic material 335 has a thickness 340 (in the direction in which optical energy is received from blazed Bragg grating 330) that is selected to increase the efficiency of emission of acoustic energy. In one example, thickness 340 is selected to be about 1/4 the acoustic wavelength of the material at the desired acoustic transmission/reception frequency. This improves the generation of acoustic energy by the photoacoustic material.

In a further example, the photoacoustic material is of a thickness 300 that is about 1/4 the acoustic wavelength of the material at the desired acoustic transmission/reception frequency, and the corresponding glass-based optical fiber imaging region resonant thickness 300 is about 1/2 the acoustic wavelength of that material at the desired acoustic transmission/reception frequency. This further improves the generation of acoustic energy by the photoacoustic material and reception of the acoustic energy by the optical fiber imaging region. A suitable photoacoustic material is pigmented polydimethylsiloxane (PDMS), such as a mixture of PDMS, carbon black, and toluene.

The imaging element described and depicted in FIGS. 3 and 4 and other varying embodiments are described in more detail in U.S. Patent Nos. 7,245.789, 7447,388, 7,660,492, 8,059,923 and in U.S. Patent Publication Nos. 2010/0087732 and 2012/0108943. In certain embodiments, an optical fiber of a imaging element (such as one shown in FIGS. 2-4) can include a plurality of Fiber Bragg Gratings, each with its own unique period (e.g., 0.5µ), that interact with at least one other transducer. Because each Fiber Bragg Grating can be directed to transmit and receive signals of specific wavelengths, the plurality of Fiber Bragg Gratings in combination with a tunable filter can be used to generate an array of distributed sonars.

Additional components may be used in conjunction with the imaging guidewire or catheter to allow an operator to image an object or surface. These additional components are referred to generally as an imaging assembly.

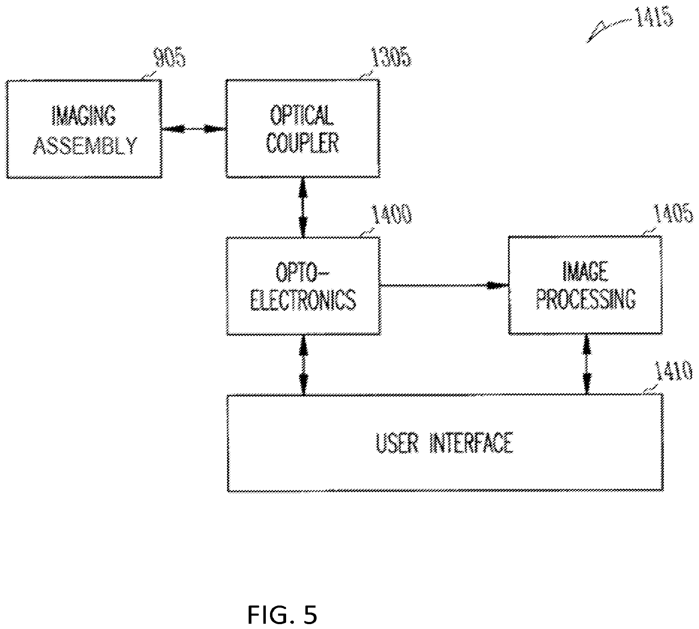

FIG. 5 is a block diagram illustrating generally an image assembly 905 and several associated interface components. The block diagram of FIG. 5 includes the image assembly 905 that is coupled by optical coupler 1305 to an optoelectronics module 1400. The optoelectronics module 1400 is coupled to an image processing module 1405 and a user interface 1410 that includes a display providing a viewable still and/or video image of the imaging region near one or more acoustic-to-optical transducers using the acoustically-modulated optical signal received therefrom. In one example, the system 1415 illustrated in the block diagram of FIG. 5 uses an image processing module 1405 and a user interface 1410 that are substantially similar to existing acoustic imaging systems.

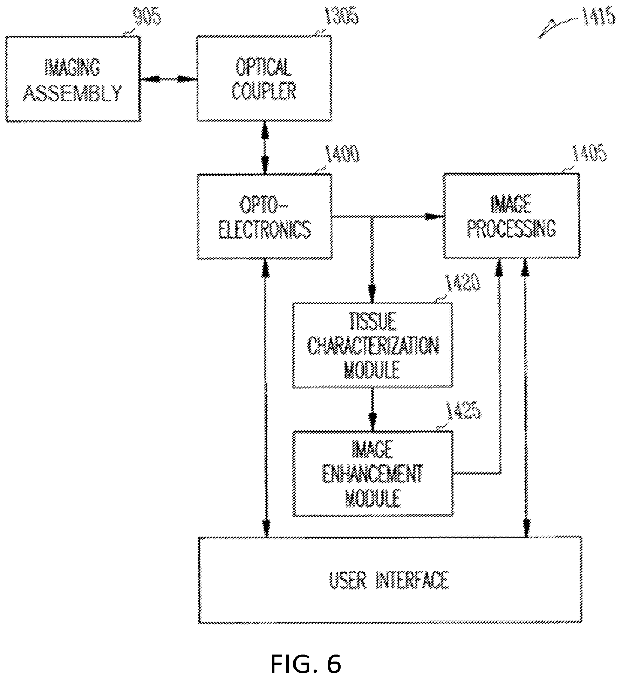

FIG. 6 is a block diagram illustrating generally another example of the image assembly 905 and associated interface components. In this example, the associated interface components include a tissue (and plaque) characterization module 1420 and an image enhancement module 1425. In this example, an input of tissue characterization module 1420 is coupled to an output from optoelectronics module 1400. An output of tissue characterization module 1420 is coupled to at least one of user interface 1410 or an input of image enhancement module 1425. An output of image enhancement module 1425 is coupled to user interface 1410, such as through image processing module 1405.

In this example, tissue characterization module 1420 processes a signal output from optoelectronics module 1400. In one example, such signal processing assists in distinguishing plaque from nearby vascular tissue. Such plaque can be conceptualized as including, among other things, cholesterol, thrombus, and loose connective tissue that build up within a blood vessel wall. Calcified plaque typically reflects ultrasound better than the nearby vascular tissue, which results in high amplitude echoes. Soft plaques, on the other hand, produce weaker and more texturally homogeneous echoes. These and other differences distinguishing between plaque deposits and nearby vascular tissue are detected using tissue characterization signal processing techniques.

For example, such tissue characterization signal processing may include performing a spectral analysis that examines the energy of the returned ultrasound signal at various frequencies. A plaque deposit will typically have a different spectral signature than nearby vascular tissue without such plaque, allowing discrimination therebetween. Such signal processing may additionally or alternatively include statistical processing (e.g., averaging, filtering, or the like) of the returned ultrasound signal in the time domain. Other signal processing techniques known in the art of tissue characterization may also be applied. In one example, the spatial distribution of the processed returned ultrasound signal is provided to image enhancement module 1425, which provides resulting image enhancement information to image processing module 1405. In this manner, image enhancement module 1425 provides information to user interface 1410 that results in a displaying plaque deposits in a visually different manner (e.g., by assigning plaque deposits a discernible color on the image) than other portions of the image. Other image enhancement techniques known in the art of imaging may also be applied. In a further example, similar techniques are used for discriminating between vulnerable plaque and other plaque, and enhancing the displayed image provides a visual indicator assisting the user in discriminating between vulnerable and other plaque.

The opto-electronics module 1400 may include one or more lasers and fiber optic elements. In one example, such as where different transmit and receive wavelengths are used, a first laser is used for providing light to the imaging assembly 905 for the transmitted ultrasound, and a separate second laser is used for providing light to the imaging assembly 905 for being modulated by the received ultrasound. In this example, a fiber optic multiplexer couples each channel (associated with a particular one of the optical fibers 925) to transmit and receive lasers and associated optics. This reduces system complexity and costs.

In one example, the sharing of transmission and reception components by multiple guidewire channels is possible at least in part because the acoustic image is acquired over a relatively short distance (e.g., millimeters). The speed of ultrasound in a human or animal is slow enough to allow for a large number of transmit/receive cycles to be performed during the time period of one image frame. For example, at an image depth (range) of about 2 cm, it will take ultrasonic energy approximately 26 microseconds to travel from the sensor to the range limit, and back. In one such example, therefore, an about 30 microseconds transmit/receive (T/R) cycle is used. In the approximately 30 milliseconds allotted to a single image frame, up to 1,000 T/R cycles can be carried out. In one example, such a large number of T/R cycles per frame allows the system to operate as a phased array even though each sensor is accessed in sequence. Such sequential access of the photoacoustic sensors in the guidewire permits (but does not require) the use of one set of T/R opto-electronics in conjunction with a sequentially operated optical multiplexer.

In certain aspects, one or more imaging elements are incorporated into the provided imaging guidewire. The imaging guidewire allows one to image a luminal surface prior to introducing a catheter into a body lumen, e.g., a blood vessel. Because the imaging guidewire obtains images of the luminal surface, an operator can use the imaging guidewire to find a region of interest within the vasculature prior to introducing a catheter device. The one or more imaging elements can be formed around an inner guidewire body, integrated into an inner guidewire body, or form the guidewire body itself. The imaging guidewire may include a support structure covering at least a portion of the imaging element. The support structure can include one or more imaging windows that allow the imaging element to send and receive signals that form the imaging data. In certain embodiments, a plurality of imaging elements surrounds an inner guidewire body. In this configuration, the imaging elements are placed next to each other, parallel to, and along the length of the inner guidewire body. The imaging elements can be optionally overlaid with a protective outer coating that provides for transmission of imaging signals.

Typically, the imaging elements are placed parallel to and along the length of the guidewire. In such aspect, the imaging elements image surfaces substantially perpendicular to the longitudinal axis of the imaging guidewire. However, other configurations may be used. For example, one or more imaging elements may be wrapped around the inner guidewire body. In addition, it is also contemplated at least a portion of the imaging elements are positioned substantially across the longitudinal axis of the guidewire. For example, the imaging elements can be positioned across a distal tip of the imaging guidewire such that the imaging elements image objects or surfaces in front of the imaging guidewire.

The imaging guidewire of the invention may be used in conjunction with any type of catheters, including delivery catheters. Furthermore, the provided imaging catheters are suitable for use with any type of guidewire. The imaging catheter allows an operator to obtain images of a luminal surface as the catheter is slideably moved along a guidewire to the location of interest. In certain embodiments, the imaging catheter is a combination catheter that can perform intraluminal procedures such as delivering implants, ablation, and extraction. Like the imaging guidewire, the provided imaging catheter includes one or more imaging elements. As discussed previously, each imaging element includes an optical fiber that may comprise a Fiber Bragg Grating. Like the imaging guidewire, the imaging elements can be positioned anywhere along and on the inner body of the imaging catheter.

In addition to the imaging functions described above, the provided guidewire also includes a pressure sensor and a flow sensor, wherein the pressure sensor and the flow sensor are fiber optic based. Pressure sensors can be used to measure pressure within the lumen and flow sensors can be used to measure the velocity of blood flow. A guidewire with both a pressure sensor and a flow sensor provides a desirable environment in which to calculate fractional flow reserve (FFR) using pressure readings, and coronary flow reserve (CFR) using flow readings.

The ability to measure and compare both the pressure and velocity flow to determine an index of hyperemic stenosis resistance significantly improves the diagnostic accuracy of ischemic testing. It has been shown that distal pressure and velocity measurements, particularly regarding the pressure drop-velocity relationship such as Fractional Flow reserve (FFR), Coronary flow reserve (CFR) and combined P-V curves, reveal information about the stenosis severity. For example, in use, the guidewire may be advanced to a location on the distal side of the stenosis. The pressure and flow velocity may then be measured at a first flow state. Then, the flow rate may be significantly increased, for example by the use of drugs such as adenosine, and the pressure and flow measured in this second, hyperemic, flow state. The pressure and flow relationships at these two flow states are then compared to assess the severity of the stenosis and provide improved guidance for any coronary interventions. The ability to take the pressure and flow measurements at the same location and same time with the combination tip sensor, improves the accuracy of these pressure-velocity loops and therefore improves the accuracy of the diagnostic information.

A pressure sensor allows one to obtain pressure measurements within a body lumen. A particular benefit of pressure sensors is that pressure sensors allow one to measure of FFR in vessel. FFR is a comparison of the pressure within a vessel at positions proximal to the stenosis and distal to the stenosis. TheFFR value represents the significance of the stenosis, which allows physicians to more accurately identify clinically relevant stenosis. For example, an FFR measurement above 0.80 indicates normal coronary blood flow and a non-significant stenosis. Another benefit is that a physician can measure the pressure before and after an intraluminal intervention procedure to determine the impact of the procedure.

A pressure sensor can be mounted on the distal portion of a flexible elongate member. In certain embodiments, the pressure sensor is positioned distal to the compressible and bendable coil segment of the elongate member. This allows the pressure sensor to move along with the coil segment as bended and away from the longitudinal axis. The pressure sensor can be formed of a crystal semiconductor material having a recess therein and forming a diaphragm bordered by a rim. A reinforcing member is bonded to the crystal and reinforces the rim of the crystal and has a cavity therein underlying the diaphragm and exposed to the diaphragm. A resistor having opposite ends is carried by the crystal and has a portion thereof overlying a portion of the diaphragm. Electrical conductor wires can be connected to opposite ends of the resistor and extend within the flexible elongate member to the proximal portion of the flexible elongate member. Additional details of suitable pressure sensors that may be used with devices of the invention are described in U.S. Pat. No. 6,106,476. U.S. Pat. No. 6,106,476 also describes suitable methods for mounting the pressure sensor 104 within a sensor housing. As discussed in further detail below, the pressure sensor can also be fiber optic-based.

The guidewire of the invention includes a flow sensor. The flow sensor can be used to measure blood flow velocity within the vessel, which can be used to assess coronary flow reserve (CFR). The flow sensor can be, for example, an ultrasound transducer, a Doppler flow sensor or any other suitable flow sensor, disposed at or in close proximity to the distal tip of the guidewire. The ultrasound transducer may be any suitable transducer, and may be mounted in the distal end using any conventional method, including the manner described in U.S. Pat. No. 5,125,137, 6,551,250 and 5,873,835. In other embodiments, however, and as explained in detail below, the flow sensor may also be fiber optic-based.

The provided guidewire may also be connected to an instrument, such as a computing device (e.g. a laptop, desktop, or tablet computer) or a physiology monitor, which converts the signals received by the sensors into pressure and velocity readings. The instrument can further calculate Coronary Flow Reserve (CFR) and Fractional Flow Reserve (FFR) and provide the readings and calculations to a user via a user interface. An exemplary device is the INSTANT WAVE-FREE RATIO device sold by Volcano Corporation.

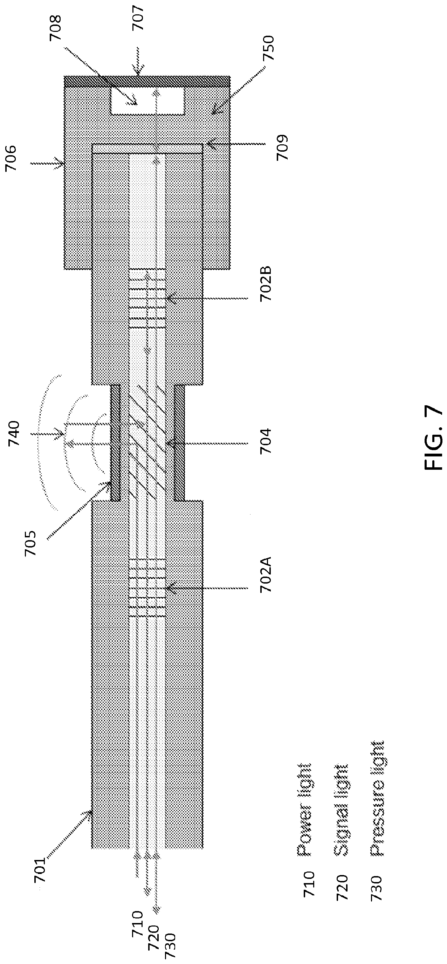

Reference will now be made to FIGS. 7-10, which depict certain exemplary embodiments of the invention. While the embodiments illustrated in these figures pertain primarily to guidewires, it is to be understood that the concepts demonstrated are equally applicable to other elongated bodies, such as catheters.

FIG. 7 depicts an exemplary optical fiber configuration for imaging as well as flow and pressure measurement. The exemplary optical fiber can be easily incorporated into a guidewire or catheter, as explained in further detail below. As shown, a single-mode optical fiber 701 is configured with multiple sensors for ultrasound (IVUS) imaging, measurement of blood flow velocity, and measurement of blood pressure. Etched into the optical fiber 701 are two unblazed FBGs 702a and 702b. Between the two unblazed FBGs 702a and 702b is a blazed FBG 704 etched into the optical fiber 701. As explained in further detail below, the blazed FBG 704 is used for ultrasound imaging as well as flow measurement. On either side of the blazed FBG is a layer of optically absorptive photoacoustic material 705 for converting light into sound waves and vice versa. At the distal end of the optical fiber 701 is a pressure sensor 706. The pressure sensor 706 includes a diaphragm 707 and a vacuum chamber 708 located behind the diaphragm 707. The pressure sensor 706 also comprises a Microelectromechanical Systems (MEMS) structure 750 and a partial mirror 709. Pressure sensors suitable for use with the invention are known in the art. One such pressure sensor, the OPP-M25, is manufactured by Opsens, Inc.

Although the provided optical fiber configuration can be of any size and dimension, in certain embodiments, the optical fiber 701 has an outer diameter of approximately 0,1524 mm (0.006") and the pressure sensor 706 has an outer diameter of up to 0,0254 mm (0.001") allowing the incorporation of the optical fiber 701 into a hollow 0,3556 mm (0,014") interventional guidewire.

Reference will now be made to the operation of the device. For ultrasound imaging, Power light 710 is supplied as pulses which match the desired frequency of ultrasound. The Power light 710 is diffracted by the blazed FBG 704 toward the optically absorptive photo acoustic material 705, which converts the light pulses into ultrasound 740. Regular FBGs transmit or reflect a percentage of or all light of certain wavelengths. Blazed FBGs, on the other hand, diffract a percentage of or all light of certain wavelengths. Ultrasound 740 is emitted from the photoacoustic material 705 and is reflected back by the structure of the blood vessel. This reflected sound is converted to signal light 720 by the photoacoustic material 705. The signal light 720 travels down the optical fiber 701 to a first unblazed FBG 702a, which reflects a portion of the signal light 720 back to the source, and second unblazed FBG 702b, which reflects the remaining signal light 720 back to the source. The ultrasound reflection from the anatomy changes the properties of the signal light passing between the two unblazed FBGs 702a and 702b, which acts as an interferometer. Externally, the modified signal light is compared to the unmodified signal light in between ultrasound pulses and the IVUS data is extracted. Further detail on the implementation of blazed FBGs in imaging devices is provided in U.S. Patent No. 7,245,789.

In the provided embodiment, pressure detection is based on a Fabry-Perot interferometer. To detect pressure, light from the source (Pressure light 730) travels down the length of the optical fiber 701 and is partially reflected by the partial mirror 709. The remaining light is continues through the MEMS structure 750 and vacuum chamber 708 and is reflected back to the source by the diaphragm 707. The pressure sensor 706 is calibrated such that a known change in the length of the light path through the vacuum chamber 708 corresponds to a specific external pressure. The light path through the vacuum chamber 708 changes as external pressure is applied to the diaphragm 707, shortening the optical path. In order to avoid interference between the imaging and pressure sensing functions, the wavelength of the pressure light 730 can be set to a different wavelength than the power light 710 discussed above or the signal light 720 to avoid interference.

In the provided embodiment, the flow function uses the same signal path and components as the imaging function described above, with different signal processing techniques. The flow function, for instance, uses a longer transmitted acoustic signal based on a longer power light pulse or multiple pulses. As signal light returns, Doppler flow signal processing is performed on the signal light 720 to measure the phase shift of the reflected ultrasound. In other embodiments, however, the flow function could be performed using a separate transmission and sensing element that functions using only diffracted light and not optoacoustic energy. Such separate flow sensors are well known in the art. See, for instance, U.S. Patent Nos. 5,125,137; 6,551,250; and 5,873,835.

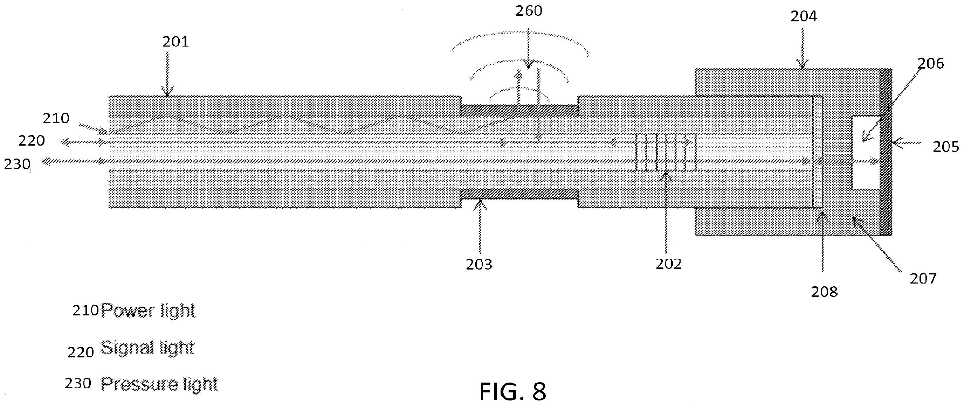

FIG. 8 depicts yet another exemplary optical fiber configuration for imaging as well as flow and pressure measurement. Again, the exemplary optical fiber can be easily incorporated into a guidewire or catheter, as explained in further detail below. As shown, a multimode, dual-core optical fiber 201 is configured with multiple sensors for ultrasound (IVUS) imaging, blood flow measurement, and blood pressure measurement. The dual core optical fiber is typically a multimode fiber with the second core being a ring around the first core and separated by cladding to isolate the light paths. Etched into the optical fiber 201 is an FBG 202. On either side of the optical fiber is a region of optically absorptive photoacoustic material 203 for converting light into sound waves and vice versa. At the distal end of the optical fiber 201 is a pressure sensor housing 204. The pressure sensor housing 204 includes a diaphragm 205 and a vacuum chamber 206 located behind the diaphragm 205. The pressure sensor 204 also comprises a MEMS structure 207 and a partial mirror 208.

Although the provided optical fiber configuration can be of any size and dimension, in certain embodiments, the optical fiber 201 has an outer diameter of approximately 0,1524 mm (0.006") and the pressure sensor 204 has an outer diameter of up to 0,0254 mm (0,001") allowing the incorporation of the optical fiber 201 into a hollow 0,3556 mm (0.014") interventional guidewire.

Reference will now be made to the operation of the device. For ultrasound imaging, Power light 210 is supplied as pulses that match the desired frequency of the ultrasound. The Power light 210 is transmitted through the outer ring core 240 and is isolated from the inner ring core 250. The Power light 210 is reflected along the outer ring core 240 and is incident upon the optically absorptive photoacoustic material 230 that converts the light pulses to ultrasound 260. Ultrasound 260 is emitted from the photoacoustic material 203 and is reflected back by the structure of the blood vessel. The signal light 220 travels down the fiber core 250 to the FBG 202, which reflects it back towards the source. The ultrasound reflection from the anatomy changes the properties of the signal light 220. Externally, the modified signal light is compared to the unmodified signal light and the IVUS data is extracted. Further detail on the use of optical elements in ultrasound imaging is provided in U.S. Patent Application No. US 2008/0114254.

In this provided embodiment, pressure measurement is again based on a Fabry-Perot interferometer. To detect pressure, light from the source (Pressure light 230) travels down the length of the optical fiber 201 and is partially reflected by the partial mirror 208. The remaining pressure light 230 continues through the MEMS structure 207 and vacuum chamber 206 and is reflected back to the source by the diaphragm 250. The pressure sensor 204 is calibrated such that a known change in the length of the light path corresponds to a specific external pressure. The light path through the vacuum chamber 206 changes as the external pressure is applied to the diaphragm 250, shortening the optical path. In order to avoid interference between the imaging and pressure sensing functions, the wavelength of the pressure light 230 can be set to a different wavelength than the power light 210 or signal light 230 to avoid interference.

In the provided embodiment, the flow function uses the same signal path and component as the imaging function described above, with different signal processing techniques. The flow function, for instance, uses a longer transmitted acoustic signal based on a longer power light pulse or multiple pulses. As signal light returns, Doppler flow signal processing is performed on the signal light 220 to measure the phase shift of the reflected ultrasound. In other embodiments, however, the flow function could be performed using a separate transmission and sensing element that functions using only diffracted light and not optoacoustic energy.

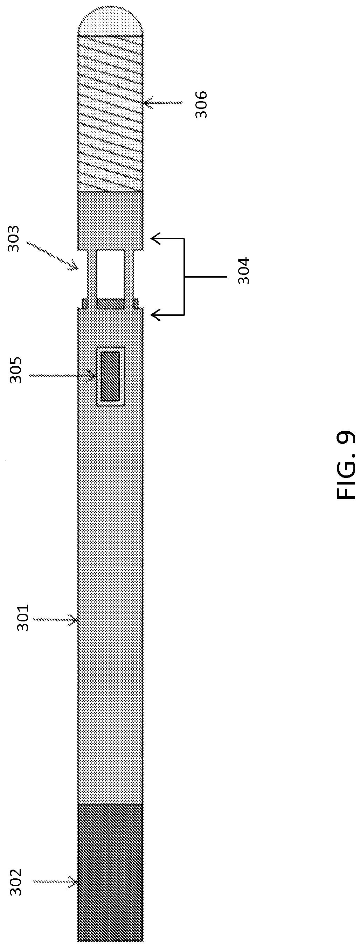

FIG. 9 illustrates an exemplary guidewire configuration for use with either fiber optic configuration described above. As shown in FIG. 9, the guidewire 300 is hollow and composed primarily of hypotubes. In the depicted embodiment, the provided guidewire 300 consists of a distal nitinol hypotube 301 joined to a proximal stainless steel hypotube 302, using conventional welding techniques. Other embodiments may use other techniques, including the use of adhesives, to join the hypotubes 301 and 302. The distal end of the nitinol hypotube 301 has a cage structure 304 comprising one or more windows 303 that allows the pressure sensor to be exposed to blood pressure. The windows 303 of the cage structure 304 can be manufactured using typical stent cutting techniques. In certain embodiments, the nitinol hypotube 301 may be slotted or cut in some other manner to improve flexibility. The nitinol hypotube 301 has one or more windows 305 at the distal end but proximal to the cage 304, that serve as openings for imaging/flow ultrasound. In certain embodiments, these windows 305 might be covered or filled with a material that is transparent to ultrasound. In further embodiments of the invention, a short radiopaque tip coil 306 is attached to the distal end of the nitinol hypotube 301 through the use of a distal core that also contributes flexibility and shape-ability to the distal end of the guidewire 300. In certain aspects of the invention, any length of the guidewire may be coated with a hydrophilic or other lubricious coating. At the proximal end of the guidewire 300, the optical fiber installed inside could terminate with a flush orientation suitable for connecting to an optical receiver or could have a variety of methods for transmitting the light signal out of the radial surface of the hypotube to an appropriate receiver.

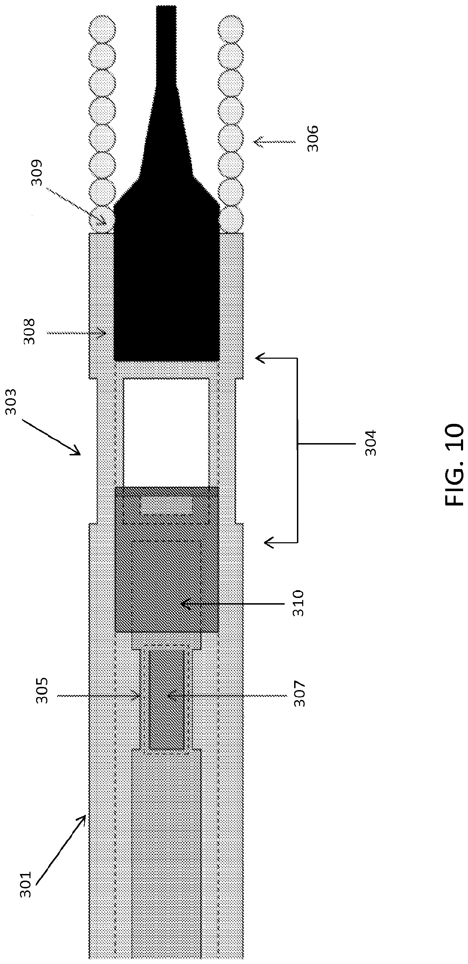

The above device can be prepared in a variety of ways. For example, a nitinol hypotube may be pre-cut with the aforementioned cage and windows, and joined to a stainless steel hypotube through convention welding. An optical fiber can be inserted through the distal end of the nitinol hypotube and aligned with the pressure sensor in the cage and the IVUS transmitter 310 located at the IVUS window. The IVUS window 305 may expose the optically absorptive photoacoustic material 307, as shown in FIG. 10. Once inserted, the optical fiber can be secured using an adhesive. Next, the tip coil can be soldered to the distal core 308 as a subassembly, as shown in FIG. 10. The tip coil subassembly is then glued onto the distal end of the nitinol hypotube 301. A shoulder 309 on the core may be used to attachment of the tip coil 306. At this stage, the entire guidewire or any portion thereof can be hydrophilically coated. It is to be noted that catheters comprising similar features may be prepared in a similar manner.

The disclosure also encompasses methods not forming part of the invention of using the provided device to assess the condition inside a vessel. The method may involve providing a device with an elongated body, such as a guidewire, inserting the device into the vessel, and using the device to assess the condition of the vessel. The device is able to image, measure pressure, and determine flow, thereby providing an assessment of the vessel condition. The contemplated devices have already been described at length throughout the present disclosure, but in short, devices uses in the provided methods incorporate optical elements into the device to operate the imaging, pressure, and flow sensors.

In practice, the method may also involve injecting a local anesthetic into the skin to numb the area of the patient prior to surgery. A puncture is then made with a needle in either the femoral artery in the groin or the radial artery in the wrist before the provided guidewire is inserted into the arterial puncture. Once positioned, the provided guidewire may then be used to ultrasonically image the vessel, measure pressure and/or flow in the vessel, techniques which all are already well-known in the art. A plastic sheath (with a stiffer plastic introducer inside it) is then threaded over the wire and pushed into the artery. The method may further involve inserting a catheter over the provided guidewire and advancing the catheter towards the heart. Once the catheter is in place, it can be used to perform a number of procedures including angioplasty, PCI (percutaneous coronary intervention) angiography, balloon septostomy, and an Electrophysiology study or ablation procedure.

## Claims

1. A system comprising an intraluminal device and a computing device, the intraluminal device comprising:
an elongate body (701, 201, 300) configured for insertion into a vessel;
an ultrasound imaging sensor (704, 202) positioned on the elongate body configured to image the interior of the vessel;
an ultrasound flow sensor (704, 262) positioned on the elongate body configured to detect a velocity of a liquid moving within the interior of the vessel;
a pressure sensor (706, 204) positioned on the elongate body configured to detect pressure in the interior of the vessel;
a single optical fiber, wherein the pressure, imaging and flow sensors are based on the single optical fiber, the elongate body is a guidewire (300), the guidewire comprises a hypotube (301, 302), a distal end of the hypotube (301) has a cage structure (304) comprising a first window (303) that allows the pressure sensor to be exposed to blood pressure, and the hypotube (301) has a second window (305) proximal to the cage structure (304), that serves as an opening for imaging and flow ultrasound, so that the optical fiber can be inserted through the hypotube to align the pressure sensor in the cage and the imaging sensor at the second window;
wherein the computing device is configured to compare pressure signals from the pressure sensor and velocity flow signals from the ultrasound flow sensor to determine an index of hyperemic stenosis resistance.

2. The device of claim 1, wherein the guidewire comprises a hollow guidewire (300).

3. The device of claim 1, wherein the optical fiber is located inside the guidewire.

4. The device of claim 1, wherein the optical fiber is a multi-core optical fiber.

5. The device of claim 1, wherein the optical fiber comprises a single mode optical fiber.

6. The device of claim 1, wherein the imaging sensor and flow sensor comprise a single sensor, which comprises a Fiber-Bragg grating (704, 202).

7. The device of claim 1, wherein the pressure sensor comprises a Fabry-Perot cavity (708, 206).

8. The device of claim 1, wherein the pressure sensor is located at a distal tip of the optical fiber.

9. The device of claim 1, wherein the guidewire comprises a hydrophilic coating.

10. The device of claim 2, wherein the guidewire comprises a nitinol hypotube (301).

11. The device of claim 1, further comprising a tip coil (306) positioned at the distal tip of the guidewire.

12. The device of claim 1, wherein the second window (305) is covered or filled with a material that is transparent to ultrasound.

## Patentansprüche

1. Ein System mit einem intraluminalen Gerät und einem Computer, wobei das intraluminale Gerät Folgendes umfasst:
einen länglichen Trägerkörper (701, 201, 300), das in ein Gefäß eingeführt wird;
einen Ultraschall-Bildgebungssensor (704, 202), der auf dem länglichen Trägerkörper positioniert ist und
das Innere des Gefäßes abbildet;
einen Ultraschall-Durchflusssensor (704, 262), der auf dem länglichen Trägerkörper positioniert ist und
die Geschwindigkeit der Flüssigkeit im Inneren des Gefäßes erfasst;
einen Drucksensor (706, 204), der auf dem länglichen Trägerkörper positioniert ist und den Druck im Inneren des Gefäßes erfasst;
einen einzelnen Lichtwellenleiter, wobei die Druck-,
Bildgebungs- und Durchflusssensoren auf dem einzelnen optischen Lichtwellenleiter beruhen,
und wobei es sich beim länglichen Trägerkörper um einen Führungsdraht (300) handelt, und wobei der Führungsdraht ein Kanülenrohr (301, 302) umfasst, und wobei das distale Ende des Kanülenrohrs (301) eine Käfigstruktur (304) aufweist, die ein erstes Fenster (303) umfasst, durch das der Drucksensor dem Blutdruck ausgesetzt werden kann, und wobei das Kanülenrohr (301) proximal zur Käfigstruktur (304) ein zweites Fenster (305) aufweist, das als Öffnung für die Bildgebung und den Strömungsultraschall dient, sodass der Lichtwellenleiter durch das Kanülenrohr eingeführt werden kann, um den Drucksensor im Käfig und
den Bildgebungssensor am zweiten Fenster auszurichten;
wobei der Computer die Drucksignale vom Drucksensor und die Flussgeschwindigkeitssignale vom Ultraschallflusssensor vergleicht, um einen Index des hyperämischen Stenosewiderstands zu ermitteln.

2. Das Gerät gemäß Anspruch 1, wobei der Führungsdraht einen hohlen Führungsdraht (300) umfasst.

3. Das Gerät gemäß Anspruch 1, wobei sich der Lichtwellenleiter im Inneren des Führungsdrahts befindet.

4. Das Gerät gemäß Anspruch 1, wobei es sich beim Lichtwellenleiter um einen mehradrigen Lichtwellenleiter handelt.

5. Das Gerät gemäß Anspruch 1, wobei der Lichtwellenleiter einen Einzelmodus-Lichtwellenleiter umfasst.

6. Das Gerät gemäß Anspruch 1, wobei der Bildgebungs- und der Durchflusssensor einen einzigen Sensor umfassen, der ein Faser-Bragg-Gitter (704, 202) umfasst.

7. Das Gerät gemäß Anspruch 1, wobei der Drucksensor einen Fabry-Perot-Hohlraum (708, 206) umfasst.

8. Das Gerät gemäß Anspruch 1, wobei sich der Drucksensor an der distalen Spitze des Lichtwellenleiter befindet.

9. Das Gerät gemäß Anspruch 1, wobei der Führungsdraht eine hydrophile Beschichtung aufweist.

10. Das Gerät gemäß Anspruch 2, wobei der Führungsdraht ein Nitinol-Kanülenrohr (301) umfasst.

11. Das Gerät gemäß Anspruch 1, das zudem eine Spitzenspule (306) umfasst, die sich an der distalen Spitze des Führungsdrahts befindet.

12. Das Gerät gemäß Anspruch 1, wobei das zweite Fenster (305) mit einem Material bedeckt oder gefüllt ist, das für Ultraschall transparent ist.

## Revendications

1. Système comprenant un dispositif intraluminal et un dispositif informatique, le dispositif intraluminal comprenant :
un corps (701, 201, 300) allongé conçu pour être inséré dans un vaisseau ;
un capteur d'imagerie (704, 202) ultrasonore positionné sur le corps allongé, conçu pour imager l'intérieur du vaisseau ;
un capteur de débit (704, 262) ultrasonore positionné sur le corps allongé, conçu pour détecter une vitesse d'un liquide se déplaçant à l'intérieur du récipient ;
un capteur de pression (706, 204) positionné sur le corps allongé, conçu pour détecter la pression à l'intérieur du vaisseau ;
une fibre optique simple, dans lequel les capteurs de pression, d'imagerie et de débit sont basés sur la fibre optique simple,
le corps allongé est un fil de guidage (300), le fil de guidage comprend un hypotube (301, 302), une extrémité distale de l'hypotube (301) comporte une structure de cage (304) comprenant une première fenêtre (303), laquelle permet au capteur de pression d'être exposé à la pression artérielle, et l'hypotube (301) comporte une seconde fenêtre (305) à proximité de la structure de cage (304), laquelle sert d'ouverture pour l'imagerie et les ultrasons de débit de telle sorte que la fibre optique peut être insérée à travers l'hypotube pour aligner le capteur de pression dans la cage et le capteur d'imagerie au niveau de la seconde fenêtre ;
dans lequel le dispositif informatique est configuré pour comparer des signaux de pression provenant du capteur de pression et des signaux de débit de vitesse provenant du capteur de débit ultrasonore pour déterminer un indice de résistance à la sténose hyperémique.

2. Dispositif selon la revendication 1, dans lequel le fil de guidage comprend un fil de guidage (300) creux.

3. Dispositif selon la revendication 1, dans lequel la fibre optique est située à l'intérieur du fil de guidage.

4. Dispositif selon la revendication 1, dans lequel la fibre optique est une fibre optique à cœurs multiples.

5. Dispositif selon la revendication 1, dans lequel la fibre optique comprend une fibre optique à mode simple.

6. Dispositif selon la revendication 1, dans lequel le capteur d'imagerie et le capteur de débit comprennent respectivement un capteur simple, lequel comprend un réseau de Bragg à fibre(704, 202).

7. Dispositif selon la revendication 1, dans lequel le capteur de pression comprend une cavité Fabry-Pérot (708, 206).

8. Dispositif selon la revendication 1, dans lequel le capteur de pression est situé à une pointe distale de la fibre optique.

9. Dispositif selon la revendication 1, dans lequel le fil de guidage comprend un revêtement hydrophile.

10. Dispositif selon la revendication 2, dans lequel le fil de guidage comprend un hypotube (301) en nitinol.

11. Dispositif selon la revendication 1, comprenant en outre une bobine de pointe (306) positionnée au niveau de la pointe distale du fil de guidage.

12. Dispositif selon la revendication 1, dans lequel la seconde fenêtre (305) est recouverte ou remplie d'un matériau transparent aux ultrasons.
